# EUROPÄISCHE PATENTANMELDUNG

(11) **EP 1 262 545 A1**
(43) Veröffentlichungstag der Anmeldung: **04.12.2002**
(21) Anmeldenummer: 01113331.1
(22) Anmeldetag: 31.05.2001
(51) Int. Cl.: C12N 15/10, B01L 3/00

(54) **Mikrostrukturen und deren Verwendung für die gerichtete Evolution von Biomolekülen**

(71) Anmelder: Direvo Biotech AG, 50829 Köln (DE)
(72) Erfinder: Koltermann, Andre, Dr., 50829 Köln (DE); Kettling, Ulrich, Dr., 50829 Köln (DE); Rarbach, Markus, Dr., 50829 Köln (DE); Stephan, Jens, Dr., 50829 Köln (DE)
(74) Vertreter: Helbing, Jörg, Dr. Dipl.-Chem.

(57) **Zusammenfassung**

Die vorliegende Erfindung betrifft Mikrostrukturen und deren Verwendung für die gerichtete Evolution von Biomolekülen.

## Beschreibung

Die vorliegende Erfindung betrifft Mikrostrukturen und deren Verwendung für die gerichtete Evolution von Biomolekülen.

In Verfahren der gerichteten molekularen Evolution werden aus Bibliotheken, die eine Vielzahl von Varianten eines Biomoleküls enthalten, Varianten selektiert, die einem festgelegten Entwicklungsziel entsprechen. Durch zyklische Wiederholung von Variation, Amplifikation und Selektion von Varianten werden optimierte Biomoleküle generiert.

Verfahren der gerichteten molekularen Evolution beruhen primär auf der Erzeugung einer großen Zahl von DNA-Varianten (Genotyp-Bibliothek). Ausgehend von einer solchen Bibliothek von Genotypen werden die korrespondierenden Genprodukte hergestellt, bzgl. Ihrer Eigenschaften (Phänotyp) durchmustert und entsprechend selektiert. Zur Untersuchung erweisen sich Screeningverfahren aufgrund ihrer Flexibilität und generellen Einsetzbarkeit gegenüber anderen, beispielsweise wachstumsgekoppelten Selektionsverfahren als besonders vorteilhaft.

Screeningverfahren basieren auf der räumlichen Isolierung der Genotypvarianten. Diese Isolierung der Genotypen sichert sowohl die getrennte Messbarkeit von Eigenschaften der unterschiedlichen Phänotypen als auch die Zuordnung von Genotyp und Phänotyp, die zur Selektion und Amplifikation optimaler Genotypen unerlässlich ist. Da die Zahl der zu untersuchenden Varianten sehr groß sein kann, wird die Segregierung der Genotypen in bisher durchgeführten Verfahren in der Regel in Probenträgern realisiert, die eine Vielzahl von Probenkompartimenten zusammenfassen. Handelsübliche Probenträger tragen beispielweise 96, 384 oder 1536 Probenkompartimente. Die Zahl der Probenkompartimente wird dabei möglichst groß gewählt, um die Zahl der benötigten Probenträger und die Menge der benötigten Assayreagenzien zu begrenzen. Limitierend in der Entwicklung von Probenträgern mit einer noch größeren Zahl von Probenkompartimenten ist die Handhabung der geringen Flüssigkeitsmengen in diesen Kompartimenten. Viskositäts- und Oberflächenspannungseinflüsse sowie Verdunstung von fluiden Proben, die in größeren Volumina von untergeordneter Bedeutung sind, beschränken diese Vorgehensweise stark.

Aufgabe der Erfindung ist es, ein Screeningverfahren für die gerichtete molekulare Evolution bereitzustellen, das die genannten Nachteile Probenträger-orientierter Screeningverfahren vermeidet.

Prinzipiell ist ein Verfahren mit vergleichbarer Aufgabe aus WO9535492 bekannt. Das beschriebene Verfahren ist geeignet, Probenbestandteile eines fluiden, in einer Kapillare transportierten Probengemisches entsprechend ihrer Eigenschaften zu trennen. Nachteilig an diesem Verfahren ist, dass keine Möglichkeit zur Reduzierung des diffusiven Stofftransports innerhalb des Fluidstroms vorgesehen ist. Dieser Mangel verhindert die Nutzung sehr kleiner Probenkompartimente, da insbesondere bei kleinen Abmessungen der diffusive Verlust von Probenbestandteilen die Durchführbarkeit der angestrebten Reaktionen im Probenkompartiment verhindert. Weiter erfordert die technische Realisierung des Verfahrens hohe Ansprüche an die mechanische Positionierung der Komponenten. Solche Lösungen erweisen sich häufig als instabil und fehleranfällig.

Es wurde gefunden, dass das Screening eines Proben-Ensembles sowie die Selektion oder Sortierung einzelner Proben aus diesem Ensemble in durch Mikrostrukturierungstechniken gefertigten Kanalstrukturen erfolgen kann. Die Aufteilung in einzelne Proben erfolgt in diesen Mikrostrukturen durch Segregierung mittels verschiedener fluider Phasen. Die vorliegende Erfindung betrifft somit
(1) Verfahren zur Selektion von Genotyp-Varianten aus Genotyp-Bibliotheken in einer Mikrostruktur, umfassend die folgende Abfolge von Reaktionsschritten:
   (a) Zusammenführen eines Testfluids, umfassend eine Genotypen-Bibliothek in einer exprimierbaren Form und geeignete Expressionshilfsstoffe, und eines Trennfluids in der Mikrostruktur, sodass einzelne Kompartimente des Testfluids entstehen;
   (b) Transportieren der Kompartimente durch die Mikrostruktur, wobei die Expression des Genotyps in den Phänotyp in den Kompartimenten erfolgt,
   (c) Detektieren des Phänotyps in den Kompartimenten und
   (d) Selektieren der Kompartimente entsprechend ihrer Phänotypen; und
(2) eine. Mikrostruktur zur Durchführung des Verfahrens nach einem der Ansprüche 1 bis 9, mit
   einem ersten Zuführkanal zum Zuführen eines Testfluids (102), insbesondere eines Genotypen enthaltenden Fluids, zu einem Reaktionskanal,
   einem zweiten Zuführkanal zum Zuführen midestens eines Trennfluids (101) zu dem Reaktionskanal,
   einer am Ende des Reaktionskanals angeordneten Detektionseinrichtung (205) zum Detektieren einer in dem Testfluid abgelaufenen Reaktion und
   einer Selektionseinrichtung zum Selektieren der Testfluid-Kompartimente (109).

Wesentliche Funktionen und Eigenschaften des erfindungsgemäßen Verfahrens (1) und der erfindungsgemäßen Mikrostruktur (2) werden im Folgenden anhand der Figuren näher erläutert. Die Figuren zeigen schematische Darstellungen unterschiedliche bevorzugte Ausführungsformen der erfindungsgemäßen Mikrostruktur.

"Mikrostruktur" im Sinne der vorliegenden Erfindung bezeichnet dreidimensionale Gebilde mit Kanalstruktur. Die Abmessungen der Kanalstrukturen liegen vorzugsweise im Bereich von 0,1 µm bis 100 µm Breite, besonders bevorzugt zwischen 1 und 10 µm. Die Aspektverhältnisse liegen vorzugsweise im Bereich von 0,1 bis 10, besonders bevorzugt bei 1.

"Fluide" im Sinne der vorliegenden Erfindung sind Flüssigkeiten oder Gase. Im einzelnen sind Testfluide zur Bildung der Genotypenkompartimente wässrige Lösungen oder Suspensionen komplexer Zusammensetzung, welche neben DNA alle weiteren Komponenten zur zellfreien in-vitro-Expression des Genotyps in dem Phänotyp enthalten.

Zur Bildung der Trennmedienkompartimente werden nicht wassermischbare Fluide eingesetzt. Dies sind insbesondere hydrophobe, inerte organisch-chemische Substanzen welche bei Betriebs-Raumtemperatur und Betriebsdruck als flüssige Phase vorliegen. In bevorzugter Form werden aliphatische oder aromatische Kohlenwasserstoffe, höhere Alkanole oder Alkanone, Ester oder Ether höherer Kohlenwasserstoffe, halogenierte Kohlenwasserstoffe, Mineralöle, Silikonöle oder Mischungen aus diesen Substanzen eingesetzt.

Als Assayfluid wird in bevorzugter Ausführungsform eine die Nachweisreagenzien enthaltende wässrige Lösung, Suspension oder Emulsion eingesetzt.

Die Flussgeschwindigkeit in den Kanälen des mikrostrukturierten Reaktionssubstrats beträgt in bevorzugter Ausführungsform zwischen 10⁻⁷ m s¹⁻ und 10⁻² m s⁻¹, in besonders bevorzugter Ausführungsform zwischen 10⁻⁶ m s⁻¹ und 10⁻⁴ m s⁻¹.

Trennung der Genotypen in einzelne Kompartimente. In bevorzugter Ausführungsform enthält jedes Kompartiment einen Genotyp. Die Segregierung der Genotypen kann dabei durch rein statistisches Verteilen oder auch gezielt durch direkte messtechnische Erfassung und entsprechende Vereinzelung der den Genotyp tragenden Biomoleküle erfolgen. Die den Genotyp tragenden Biomoleküle bestehen in der Regel aus doppelsträngiger DNA, ggfs. auch aus einzelsträngiger DNA oder RNA. In einer weiteren Ausführungsform werden mehrere Genotypen in einem Kompartiment zusammengefasst, um eine höhere Variantentenzahl untersuchen zu können. Ausgehend vom Genotyp wird innerhalb des Kompartiments der entsprechende Phänotyp gebildet. Die Segregierung erfüllt dabei sowohl die Aufgabe der Trennung der Vielzahl von Phänotypen zur Messung der Eigenschaften als auch der Kopplung von Genotyp und Phänotyp, welche eine anschliessende Isolierung verbesserter Genotypen ermöglicht. Die Kompartimentvolumina liegen allgemein im Bereich zwischen 0,01 und 10.000 fl, vorzugsweise im Bereich zwischen 0,1 und 1000 fl, besonders bevorzugt zwischen 1 und 100 fl.

Zugabe von Assayreagenzien in die einzelnen Kompartimente. Geeignete Reagenzien zur Detektion des Phänotyps können in der Mehrzahl der Anwendungen nicht bei der Bildung der Genotyp-Kompartimente zugegeben werden, da diese zum einen mit in-vitro-Verfahren zur Expression des Phänotyps interferieren können und da andererseits eine exakte zeitliche Kontrolle des Reaktionsverlaufs durch die frühe Zugabe der Reagenzien erschwert wird.

Detektion des Phänotyps. Die Bestimmung der phänotypischen Eigenschaften jeden Genotyps bzw. jedes Kompartiments erfolgt vorzugsweise mit optischen, besonders bevorzugt mit fluorimetrischen Verfahren. Geeignete Messverfahren zur Messung in Strukturabmessungen bis zu wenigen 100 nm sind beispielsweise beschrieben in DE 197 57 740.

Die Auslese von Genotypen. Die Auslese der Genotypen mit positiv beurteiltem Phänotyp wird primär durch Selektion der entsprechenden Kompartimente erreicht. Der Genotyp wird in Form der DNA oder RNA aus den einzelnen selektierten Kompartimenten isoliert und dem Prozess erneut zu geführt.

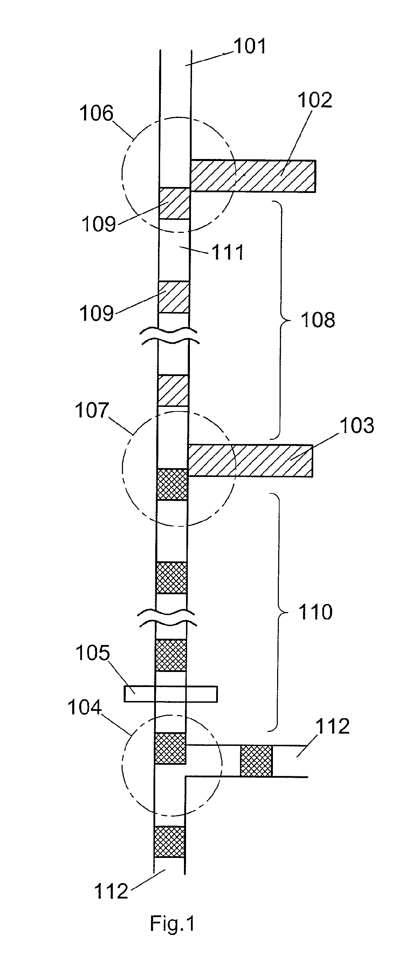

Schematisch ist der Aufbau einer mikrostrukturierten Kanalstruktur, weche diese Funktionen ineinander vereint und so die gerichtete Evolution von Biomolekülen ermöglicht, in Fig. 1 wiedergegeben.

Die Trennung der Kompartimente zur Segregierung der Genotypen erfolgt durch intermittierende Zugabe mindestens eines die Genotypen enthaltenden Testfluids (102) und mindestens eines Trennfluids (101) in einer Kompartimentierungsstruktur (106). Das Fluid (102) enthält dabei vorzugsweise alle zur Expression des Genotyps notwendigen Substanzen, die Zusammensetzung solcher Fluide und Verfahren zu deren Herstellung sind dem Fachmann bekannt (Lesley, S. A., Methods Mol. Biol. 37, 265 (1995)). Das Trennfluid kann sowohl eine wässrige Lösung als auch eine nicht-wässrige, vorzugsweise nicht wassermischbare Flüssigkeit oder ein Gas sein. Auch können zur Trennung der Genotypkompartimente (109) in sich strukturierte Trennfluidkompartimente (111) verwendet werden, die die Zugabe mehrerer Trennfluidkomponenten notwendig machen.
Die Temperaturen der beiden Reaktionsbereiche I (108) und II (110) können dabei durch geeignete Thermostatisierungselemente gesteuert werden. Beide Reaktionsbereiche (108 und 110) können anwendungsabhängig bei gleicher oder unterschiedlicher Temperatur betrieben werden.
Die Verwendung nicht wassermischbarer Fluide oder Gase als Trennfluid (101) in Kombination mit hydrodynamischen Fluss im mikrostrukturierten Reaktionssubstrat ist vorteilhaft, da durch diese Segregierung der Genotypkompartimente diffusiver Stofftransport zwischen Genotypkompartimenten (109) und zwischen Genotypkompartimenten (109) und Trennfluid (101) minimiert werden kann. Weiter ist vorteilhaft, dass durch die Verwendung nicht wassermischbarer Fluide oder Gase als Trennfluid (101) im hydrodynamischen Fluss die Axialdispersion minimiert wird. Die Expression des Genotyp erfolgt im Reaktionsbereich I (108). Die Reaktionszeit kann durch Experimentator durch Wahl der Länge des mikrostrukturierten Reaktionskanals im Reaktionsbereich I sowie durch Wahl der Fluidgeschwindigkeit im Reaktionsbereich I frei gewählt werden. Die Zugabe von Reaktionskomponenten zur Bestimmung phänotypischer Eigenschaften der im Genotypkompartiment (109) im Reaktionsbereich (108) gebildeten Biomoleküle wird in einem Bereich (107) des mikrostrukturierten Substrats eine durch den Experimentator gewählte Menge eines Assay-Fluids (103) mit den Genotypkompartimenten (109) vereinigt. Vorzugsweise besteht das Assay-Fluid (103) aus einem Fluid, das mit dem Genotypkompartimenten (109) mischbar oder in diesem löslich ist. Die Umsetzung der mit dem Assayfluid (103) zugesetzten Reaktionskomponenten durch die im Genotypkompartimenten (109) vorliegenden Biomoleküle erfolgt im Reaktionsbereich II (110). Die Reaktionszeit kann durch den Experimentator durch Wahl der Länge des mikrostrukturierten Reaktionskanals im Reaktionsbereich II sowie durch Wahl der Fluidgeschwindigkeit im Reaktionsbereich II frei gewählt werden.

Die Messung der aus der Umsetzung der Komponenten aus Assayfluid (103) und Genotypkompartiment (109) hervorgehenden Reaktionsprodukte erfolgt in einem Messbereich (105) des Reaktionssubstrats. Vorzugsweise werden zur Messung spektroskopische Messverfahren, höchst vorzugsweise konfokale fluoreszenzspektroskopische Methoden eingesetzt. Solche Methoden sind in der Lage in Strukturenabmessungen von wenigen 100 nm mit hoher Sensitivität die Probenzusammensetzung zu bestimmen. Die Anwendung konfokaler Detektionsverfahren zur Detektion kleinster Substanzmengen ist beispielweise in DE4301005 und WO9535492 gezeigt.

Genotypkompartimente mit im Sinne des Entwicklungsziels positiven Messergebnissen müssen von solchen mit negativen Messergebnissen getrennt werden, um vorteilhafte Varianten der eingesetzten Bibliothek von nachteiligen zu trennen. Diese Trennung erfolgt in einem Selektionsbereich (104) des Reaktionssubstrats durch gesteuerte Lenkung der Genotypkompartimente in einen von mindestens zwei Selektionskanälen 112 und 113.

Alle genannten Prozessschritte werden in durch Mikrostrukturtechnik gefertigten Kanalstrukturen vorteilhaft zusammengeführt und integriert. Derartige Kanalstrukturen können aus verschiedenen Werkstoffen gefertigt werden. Dazu zählen Metalle (z.B. Silizium), amorphe Werkstoffe (z.B. Glas), keramische Werkstoffe und polymere Werkstoffe (z.B. Polyurethane (PU) und Polydimethylsiloxane (PDMS)). Bevorzugt werden die Kanalstrukturen durch Abscheidungs- oder Abtragungstechniken in metallischen, keramischen oder amorphen Materialien hergestellt. Vorteile dieser Ausführungsformen sind die geringen erreichbaren Toleranzen der Strukturen sowie eine hohe Funktionalität der integrierbaren Bauelemente. Daneben erlauben softlithografische Verfahren und Abformungsverfahren die Herstellung von mikrostrukturierten Reaktionssubstraten aus polymeren Materialien wie Polyurethanen (PU) und Polydimethylsiloxanen (PDMS). Da integrierte Funktionselemente innerhalb mikrostrukturierter Reaktionssubstrate gegeneinander fixiert sind ergeben sich hier gegenüber anderen Lösungsansätzen wesentliche Vorteile in Bezug auf die Stabilität des Systems.

Aktive Bauelemente können als Bestandteil der Mikrostruktur als Formgedächtniselemente, piezoelektrische Baugruppen oder magnetostriktive Elemente ausgeführt werden. In bevorzugter Ausführung werden aktive Bauelemente als Formgedächtniselemente ausgeführt. Zu den integrierbaren Bauelementen zählen beispielsweise Ventile und Pumpen [Gerlach T. Schuenemann M. Wurmus H.; Journal of Micromechanics & Microengineering. 5(2):199-201, 1995 Jun.].

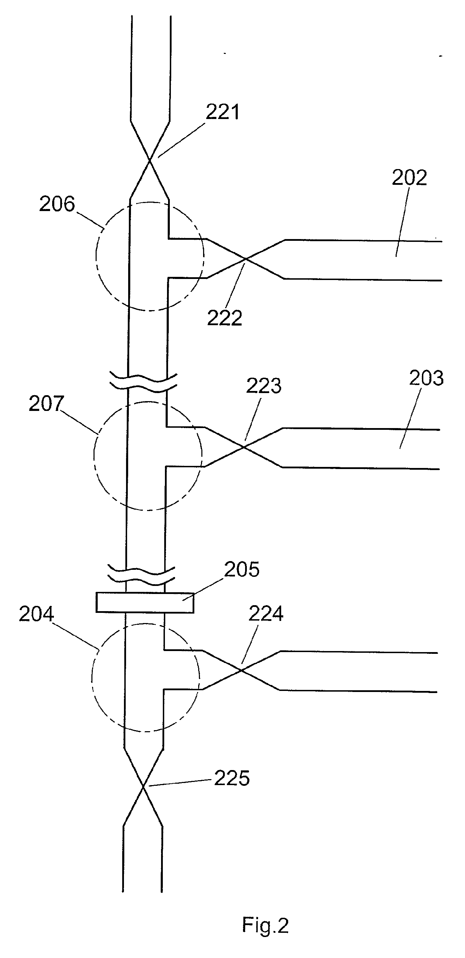

Aktive Bauelemente werden in bevorzugter Ausführungsform zur Segregierung im Fluidstrom und zur Selektion ausgewählter Kompartimente eingesetzt. Der funktionale Aufbau einer erfindungsgemäßen mikrostrukturierten Kanalstruktur mit aktiven Bauelementen 221, 222, 223, 224 und 225 ist in Fig. 2 wiedergegeben. Die mikrostrukturierten Ventilelemente 221 und 222 dienen zur Bildung des beschriebenen kompartimentierten Fluidstroms aus den Fluidkomponenten 201 und 202 im Kompartimentierungselement 206. Die Ventilelemente werden in bevorzugter Ausführungsform alternierend für eine durch den Experimentator festgelegte Zeitspanne geöffnet und gestatten damit den Durchtritt von Fluidelementen definierten Volumens.

Das Ventilelement 223 steuert die Zugabe des Assayfluid 203 im Bereich 207. In bevorzugter Ausführungsform wird die Steuerung des Ventilelements mit der Steuerung der Ventilelemente 221 und 222 so koordiniert, dass Assayreagenzien dann zugegeben werden, wenn sich ein Genotypenkompartiment innerhalb des Zugabebereiches 207 befindet. Insbesondere bei Verwendung kompressible Fluide oder mikrostrukturierte Reaktionssubstrate aus elastischen Materialien kann die Koordinierung der Ventilelemente 221, 222 und 223 unzureichend sein, um die Zugabe der Assayreagenzien zu einem Genotypenkompartiment im Zugabebereich 207 sicher zu gewährleisten. In weiterer bevorzugter Ausführungsform kann dann der Transport eines Gentotypenkompartiments in den Zugabebereich 207 messtechnisch bestimmt und das Ventilelement 223 ausgehend von diesem Meßwert geöffnet werden. Bevorzugt werden zur Detektion des Gentotypenkompartiments im Zugabebereich 207 optische Messverfahren eingesetzt.

In einer weiteren bevorzugten Ausführungsform können phänotypische Eigenschaften des gebildeten Genprodukts in direkter Umgebung des Assayfluidzugabebereichs (507) bestimmt werden. Das erfindungsgemäße mikrostrukturierte Reaktionssubstrat wird unter Auslassung des Reaktionsbereichs II (410) dann so hergestellt, dass Assayfluidzugabebereich (507) und Detektionsbereich (505) örtlich zusammenfallen. Vorteile dieser Ausführungsform sind, dass schnelle Umsetzungen zwischen Genprodukt und Assayreagenzien so als zeitlich aufgelöste Messreihen beobachtet werden können. Solche Zeitreihenmessungen gestatten für schnelle Umsetzungen eine bessere Charakterisierung phänotypischer Eigenschaften des gebildeten Genprodukts als die Bestimmung eines einzelnen Messwerts nach einer durch die Wahl der Länge des Reaktionsbereichs II gegebenen festgelegten Reaktionszeit. Die Dauer der Zeitreihenmessung kann anwendungsabhängig durch Unterbrechung des Fluidstroms im mikrostrukturierten Reaktionssubstrat durch gleichzeitiges Schließen der Ventilelemente 521 und 522 verlängert werden.

Die Ventilelemente 224 und 225 steuern die Selektion der Genotypenkompartimente nach Bestimmung deren phänotypischer Eigenschaften im Detektionsbereich 205. Zur Selektion einzelner Genotypenkompartimente werden die Ventilelemente der mindestens zwei Selektionskanäle 212 und 213 alternativ geöffnet.

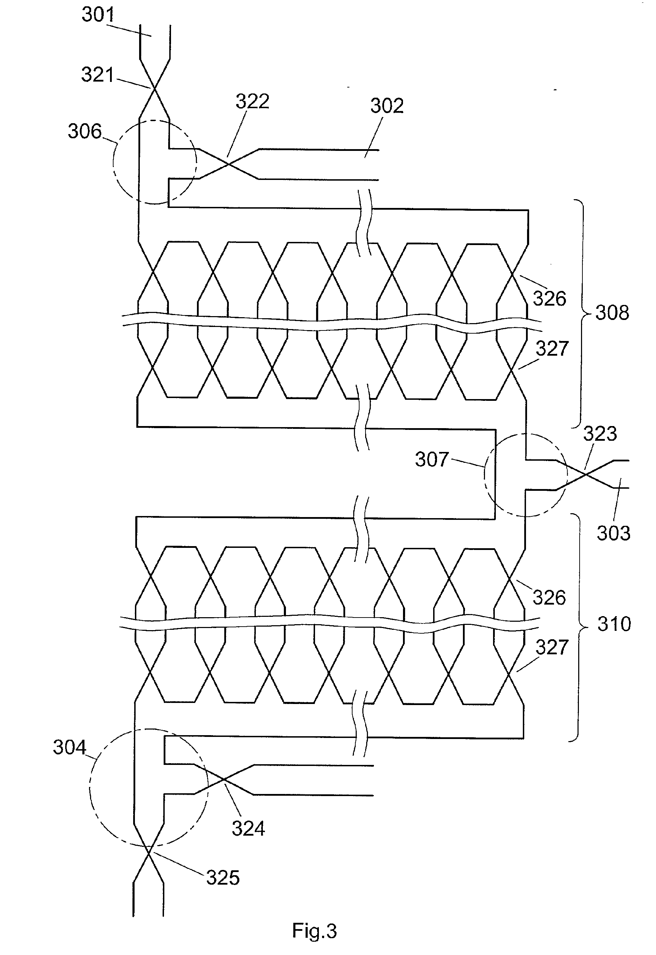

Die Aufenthaltszeit von Probenkompartimenten in den Reaktionsbereichen 108 und 110 des in Fig. 1 schematisch dargestellten Reaktionssubstrats ist gegeben durch die Länge des Reaktionsbereichs und die Fluidgeschwindigkeit in diesem Bereich. Nach dem Fachmann bekannten Zusammenhängen steigt bei hydrodynamischen Fluidtransport der Druckverlust mit der Länge der Kanalstruktur. Für sehr lange Reaktionszeiten und den damit notwendigen langen Reaktionsbereichen kann der zum hydrodynamischen Transport erforderliche Druck ein technisches Hemmnis zur Konstruktion des erfindungsgemäßen mikrostrukturierten Reaktionssubstrats sein. In diesem Fall stellt die in Fig. 3 skizzierte Ausführungsform eine vorteilhafte Lösung des technischen Problems dar. Die Reaktionsbereiche 308 und 310 sind hier ausgeführt als getrennte, durch Öffnen einzelner Ventilelemente 326 ansteuerbare Reaktionskanäle 327. Im erfindungsgemäßen Verfahren werden so einzelne Kanäle nach Öffnen einzelner Ventile mit dem kompartimentierten Fluidstrom befüllt. Die Vielzahl vorgesehener Reaktionskanäle 326 kann so befüllt werden. Nach Ablauf einer frei wählbaren Reaktionszeit kann das in den einzelnen Reaktionskanälen vorliegende kompartimentierte Fluid durch weiteres kompartimentiertes Fluid oder durch ein nicht kompartimentiertes Fluid verdrängt und dem jeweils nächsten Funktionselement (307) oder (304) zugeleitet werden.

Erfindungsgemäß ist das Verfahren geeignet, Biomoleküle mit bestimmten phänotypische Eigenschaften aus einer Vielzahl von Varianten zu selektiern. Die Varianten können durch in-vitro-Verfahren zur Mutation einer DNA-Sequenz des Ausgangsphänotyps hergestellt werden. Besonders ist das Verfahren für die Selektion von Phänotypen mit nicht zellkompatiblen Phänotypen geeignet. Nicht zellkompatibel ist beispielsweise eine sequenzspezifische Endonukleaseaktivität, wenn der Zelle die Methylaseaktivität mit entsprechender Sequenzspezifität fehlt.

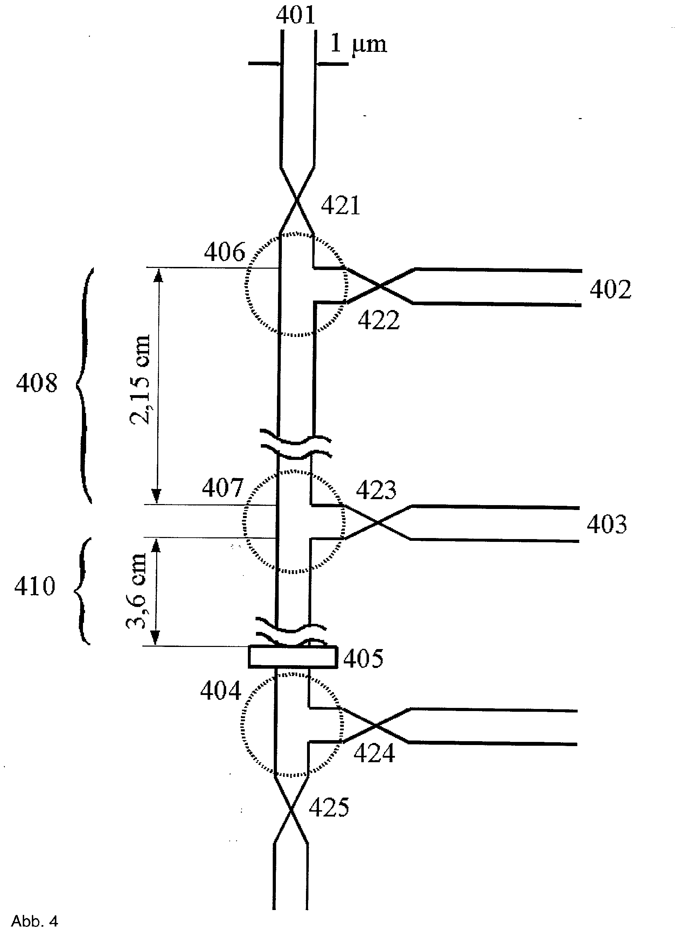

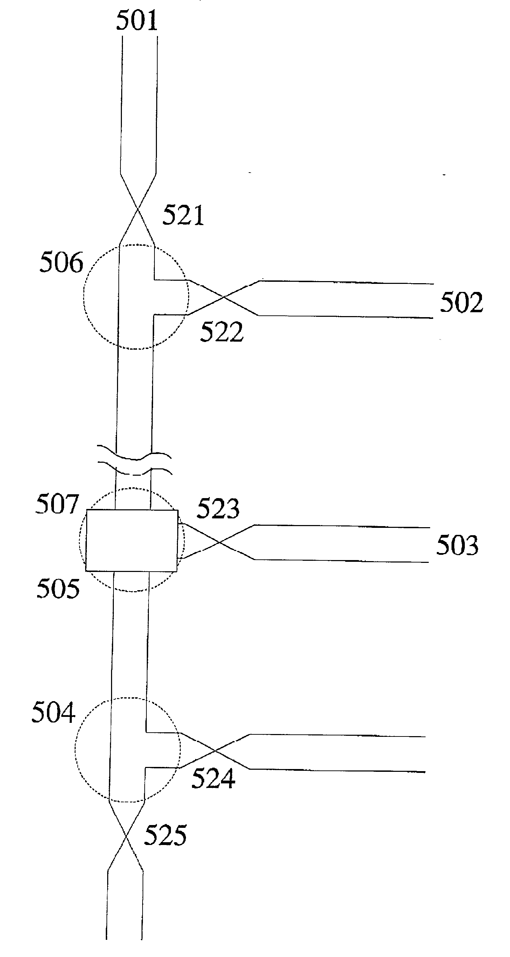

Für die Selektion einer spequenzspezifischen Endonukleaseaktivität ist ein Mikrostruktur-Design entsprechend Fig. 4 zu wählen. Die Kanalbreite und - tiefe soll etwa 1 µm betragen. Das Expressionsfluid (402) enthält einen zur zellfreien Proteinexpression geeigneten Zellextrakt. Vorzugsweise wird zur zeitlich gekoppelten in-vitro-Transkription/Translation ein E.-coli-S30-Extrakt verwendet (Lesley, S.A., Methods Mol. Biol. 37, 265 (1995)). Die Genotypenbibliothek wird in diesem Ansatz bei einer Temperatur von 4 °C auf eine Konzentration von 500 pM Plasmid-DNA verdünnt. Als Trennfluid (410) wird niederviskoses Mineralöl verwendet. Die Ventilelemente (421 und 422) des Kompartimentierungselements (406) des mikrostrukturierten Reaktionssubstrats werden bei Zugabe von Trennfluid (402) und Trennfluid (401) so gesteuert, dass sich wässrige Genotypkompartimente mit einer Länge von ca. 2 µm und Trenfluidkompartimente mit einer Länge von 10 µm bilden. In Kanalabmessungen von 1 µm x 1 µm beträgt das Volumen eines einzelnen Genotypenkompartiments entsprechend 2 fl, jedes Genotypenkompartiment trägt dann im statistischen Mittel ca. 0,6 DNA-Moleküle der eingesetzten Bibliothek. Entsprechend enthalten ca. 54 % der gebildeten Kompartimente kein DNA-Molekül, 33 % der Kompartimente ein DNA-Molekül und 13 % der Kompartimente zwei oder mehr DNA-Moleküle. Es wurde gefunden, dass es durch unspezifische nukleolytische Aktivität häufig zu einem Verlust an eingesetzter DNA kommt, sodass die Konzentration der eingesetzten DNA anwendungsabhängig geringfügig höher im nanomolaren (1 - 10 nM) Bereich gewählt werden kann. Die Transportgeschwindigkeit innerhalb des Inkubationsbereichs I (408) der Kanalstruktur wird mit ca. 4,3 cm h⁻¹ gewählt, sodass jedes gebildete Genotoypenkompartiment nach ca. 0,5 h die Inkubationsstrecke I von 2,15 cm zur Proteinexpression durchlaufen hat. Nach Expression des Phänotyps werden den Genotypenkompartimenten jeweils ca. 8 fl Assayfluids (403) zudosiert. Das Assayfluid enthält alle für die endonukleolytische Reaktion und ihren Nachweis nötigen Komponenten. Folgende Zusammensetzungen des Assayfluids (403) sind hierfür geeignet: 150 mM KOAc, 37,5 mM Tris-Acetat, ph 7,6, 15 mM MgOAc, 0,75 mM β-Mercaptoethanol, 515 µg/ml BSA, 0,05 % Triton X-100, 0,5 % Glycerin, 10 nM doppelt fluoreszenzgelabeltes DNA-Substrat. Entsprechend den in DE 19757740 genannten Methoden kann die katalytische Aktivität einer Endonuklease spezifisch durch Zusatz doppelt fluoreszenzmarkeirter DNA-Substrate bestimmt werden. Durch die Zugabe des Assayfluids (403) erhöht sich die mittlere Fluidgeschwindigkeit auf 7,2 cm h⁻¹. Nach einer Inkubationsszeit von 0,5 h durchläuft jedes Genotypenkompartiment den Inkubationsbereich II (410) von 3,6 cm Länge zum Detektionselement (405). Entsprechend den in DE 197 57 740 genannten Methoden kann die katalytische Aktivität einer Endonuklease spezifisch über fluoreszenzspektroskopische Verfahren nachgewiesen werden. Am Selektionselement (404) erfolgt dann durch geeignete Steuerung der Ventilelemente (424 und 425) die Selektion positiv bewerteter Genotypenkompartimente.

## Patentansprüche

1. Verfahren zur Selektion von Genotyp-Varianten aus Genotyp-Bibliotheken in einer Mikrostruktur, umfassend die folgende Abfolge von Reaktionsschritten:
(a) Zusammenführen eines Testfluids, umfassend eine Genotypen-Bibliothek in einer exprimierbaren Form und geeignete Expressionshilfsstoffe, und eines Trennfluids in der Mikrostruktur, sodass einzelne Kompartimente des Testfluids entstehen;
(b) Transportieren der Kompartimente durch die Mikrostruktur, wobei die Expression des Genotyps in den Phänotyp in den Kompartimenten erfolgt,
(c) Detektieren des Phänotyps in den Kompartimenten und
(d) Selektieren der Kompartimente entsprechend ihrer Phänotypen.

2. Verfahren nach Anspruch 1, wobei nach Schritt (b) ein Assayfluid mit Reagenzien, die zum Nachweis des Phänotyps geeignet sind, zu den Kompartimenten zugegeben wird.

3. Verfahren nach Anspruch 1 oder 2, wobei
- das Testfluid und geeignete Expressionshilfsstoffe, ausgewählt sind aus wässrigen Lösungen und Suspensionen komplexer Zusammensetzungen und/oder
- das Trennfluid ein nicht wassermischbares Fluid ist und insbesondere ausgewählt ist aus aliphatischen und aromatischen Kohlenwasserstoffen, höheren Alkoholen, höheren Alkanonen, Estern und Ethern höherer Kohlenwasserstoffe, halogeniden Kohlenwasserstoffen und Silikonen und Mischungen aus diesen Substanzen und/oder
- die Transportgeschwindigkeit der Kompartimente in der Mikrostruktur 1 x 10⁻⁷ bis 1 x 10⁻² m/s, vorzugsweise 1 x 10⁻⁶ bis 1 x 10⁻⁴ m/s beträgt.

4. Verfahren nach einem oder mehreren der Ansprüche 1 bis 3, wobei die Konzentration der Genotypen-Bibliothek und das Zusammenführen von Testfluid und Trennfluid so gewählt sind, dass im statistischen Mittel nur eine Genotypvariante pro Kompartiment enthalten ist, und/oder wobei das Kompartimentvolumen 0,01 fl bis 10 pl, vorzugsweise 0,1 fl bis 1 pl, besonders bevorzugt bis 100 fl beträgt.

5. Verfahren nach einem oder mehreren der Ansprüche 2 bis 4, wobei das Assayfluid mit dem Testfluid mischbar und mit dem Trennfluid unmischbar ist, insbesondere ausgewählt ist aus wässrigen Lösungen, Suspensionen und Emulsionen, und der Zeitpunkt der Zugabe so gewählt ist, dass sich bereits eine für die Detektion hinreichende Menge Genprodukt gebildet hat.

6. Verfahren nach einem oder mehreren der Ansprüche 1 bis 5, wobei
- die Assayreagenzien für die zu selektierende Funktion spezifisch sind und insbesondere solche sind, die geeignet sind, die zu selektierende Funktion mit optischen, vorzugsweise fluorimetrischen Messverfahren zu analysieren und/oder
- das Detektieren des Phänotyps in den Kompartimenten die qualitative und/oder quantitative Bestimmung der phänotypischen Eigenschaften umfasst, und insbesondere durch optische, besonders bevorzugt durch fluorimetrische Verfahren erfolgt.

7. Verfahren nach einem oder mehreren der Ansprüche 1 bis 6, wobei der Phänotyp endonukleolytische Aktivität ist.

8. Verfahren nach einem oder mehreren der Ansprüche 1 bis 7, wobei das Selektieren der Kompartimente durch Sortieren erfolgt und/oder das Verfahren weiterhin den Reaktionsschritt
(e) Isolieren des Genotyps der aussortierten Kompartimente unter Bildung einer neuen Genotyp-Bibliothek umfasst.

9. Verfahren nach Anspruch 8, wobei die erhaltene Genotyp-Bibliothek einem oder mehreren weiteren Reaktionszyklen (a) bis (d) unterworfen wird.

10. Mikrostruktur zur Durchführung des Verfahrens nach einem der Ansprüche 1 bis 9, mit
einem ersten Zuführkanal zum Zuführen eines Testfluids (102), insbesondere eines Genotypen enthaltenden Fluids, zu einem Reaktionskanal,
einem zweiten Zuführkanal zum Zuführen mindestens eines Trennfluids (101) zu dem Reaktionskanal,
einer am Ende des Reaktionskanals angeordneten Detektionseinrichtung (205) zum Detektieren einer in dem Testfluid abgelaufenen Reaktion und
einer Selektionseinrichtung zum Selektieren der Testfluid-Kompartimente (109).

11. Mikrostruktur nach Anspruch 10, **gekennzeichnet durch** eine erste, in dem ersten oder zweiten Zuführkanal angeordneten Dosiereinrichtung (221, 222) zur volumenbegrenzten Zufuhr von Testfluid (102) bzw. Trennfluid (101), so dass in dem Reaktionskanal Kompartimente (109, 111) ausgebildet sind.

12. Mikrostruktur nach Anspruch 11, **gekennzeichnet durch** eine zweite Dosiereinrichtung (222), die derart angeordnet ist, dass im ersten und zweiten Zuführkanal je eine Dosiereinrichtung (221, 222) vorgesehen ist.

13. Mikrostruktur nach Anspruch 12, **gekennzeichnet durch** eine mit den Dosiereinrichtungen (221, 222) verbundene Steuereinrichtung, **durch** die die Dosiereinrichtungen (221, 222) derart steuerbar sind, dass abwechselnd nur Testfluid (102) und Trennfluid (101) dem Reaktionskanal zugeführt werden.

14. Mikrostruktur nach einem der Ansprüche 10 - 13, **gekennzeichnet durch** einen dritten Zuführkanal zum Zuführen von Assayfluid (103) in den Reaktionskanal.

15. Mikrostruktur nach Anspruch 14, **dadurch gekennzeichnet, dass** in dem dritten Zuführkanal eine dritte Dosiereinrichtung (223) zur volumenbegrenzten Zufuhr von Assayfluid (103) vorgesehen ist, die mit einer Erkennungseinrichtung zum Erkennen eines Testfluid-Kompartiment (109) verbunden ist und in Abhängigkeit eines von der Erkennungseinrichtung abgegebenen Signals steuerbar ist, so dass das Assayfluid (103) dem Testfluid-Kompartiment (109) zugeführt wird.

16. Mikrostruktur nach einem der Ansprüche 10 - 15, **dadurch gekennzeichnet, dass** die Selektionseinrichtung mindestens zwei mit dem Reaktionskanal verbundene Selektionskanäle (112) und eine Auswahleinrichtung (224, 225) zur Auswahl eines der beiden Selektionskanäle (112) in Abhängigkeit des Detektionsergebnisses aufweist.

17. Mikrostruktur nach Anspruch 16, **dadurch gekennzeichnet, dass** als Selektionseinrichtung in mindestens einem der Selektionskanäle (112) eine Dosiereinrichtung (224, 225) vorgesehen ist.

18. Mikrostruktur nach einem der Ansprüche 10 - 17, **dadurch gekennzeichnet, dass** der Reaktionskanal (108, 110) mehrere zueinander parallel schaltbare Einzelkanäle (308, 310) aufweisen.

19. Mikrostruktur nach Anspruch 19, **dadurch gekennzeichnet, dass** jeder Einzelkanal mindestens eine Einlass-Dosiereinrichtung und/oder eine Auslass-Dosiereinrichtung aufweist.

20. Mikrostruktur nach einem der Ansprüche 10 - 20, **dadurch gekennzeichnet, dass** die Dosiereinrichtungen (222, 223, 224) mikrostukturierte Ventilelemente sind.
